# EUROPEAN PATENT SPECIFICATION

(11) **EP 0 680 356 B1**
(45) Date of publication and mention of the grant of the patent: **12.04.2000**
(21) Application number: 93904520.9
(22) Date of filing: 05.01.1993
(51) Int. Cl.: A61M 25/06

(54) **CATHETER INTRODUCER**
KATHETEREINFÜHRVORRICHTUNG
INTRODUCTEUR DE CATHETER

(30) Priority: 07.01.1992 JP 16192 U; 29.01.1992 JP 282692 U
(43) Date of publication of application: 08.11.1995
(73) Proprietor: Sherwood Services AG, 8200 Schaffhausen (CH)
(72) Inventor: OKADA, Yosuke, Shyuchi-gun, Shizuoka Prefecture (JP); KURIMOTO, Munehito, Nippon Sherwood Med. Ind.,Ltd, Sendagaya, Shibuya-ku (JP)
(74) Representative: Rüedi, Regula Béatrice
(86) International application number: US9300437
(87) International publication number: WO9313822

(56) References cited:
- EP-A- 0 008 451
- EP-A- 0 171 077
- EP-A- 0 258 566
- US-A- 3 406 685
- US-A- 4 772 266

## Description

### TECHNICAL FIELD

The present device relates to a catheter introducer for introducing a catheter into a blood vessel.

### BACKGROUND ART

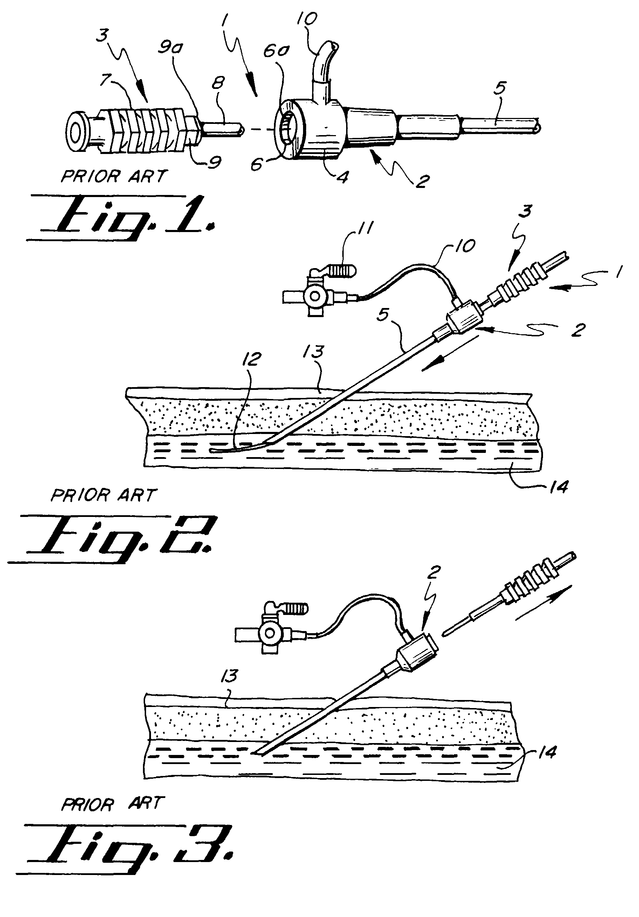

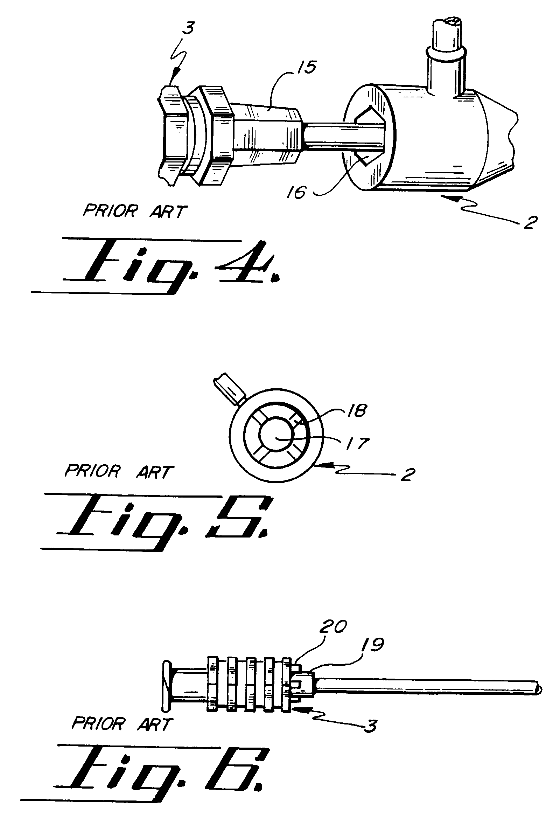

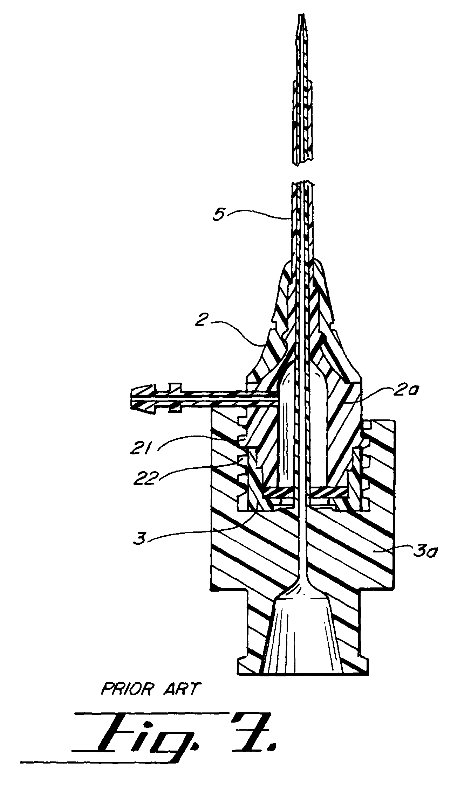

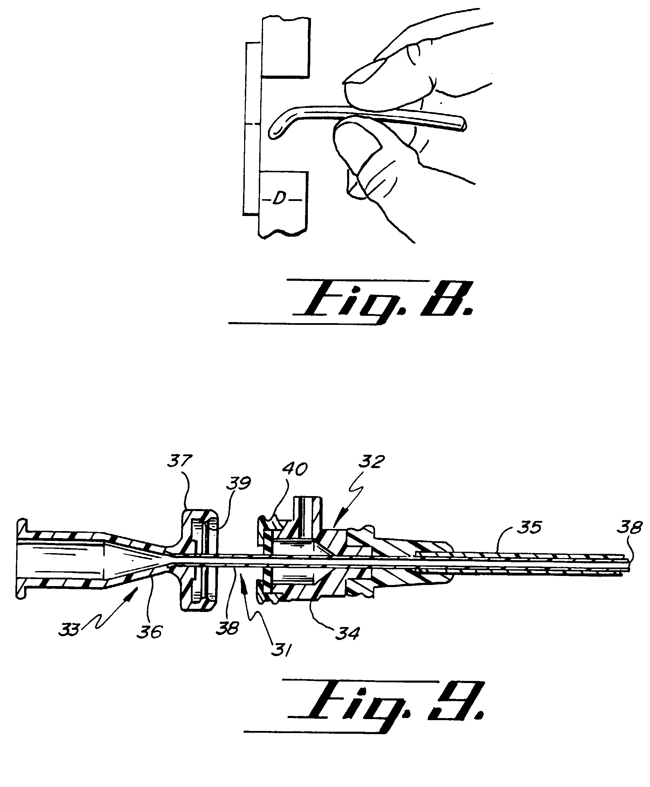

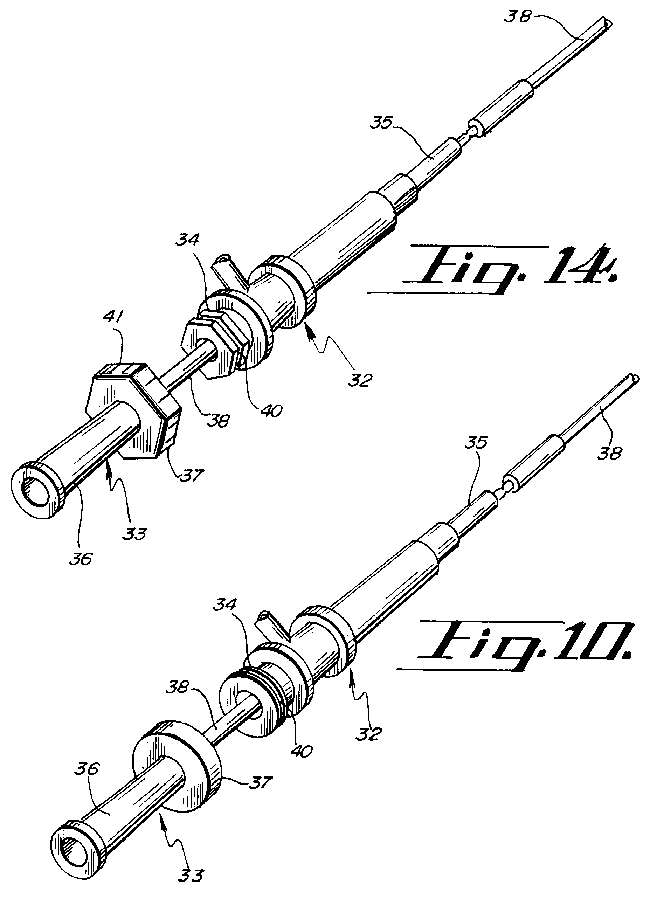

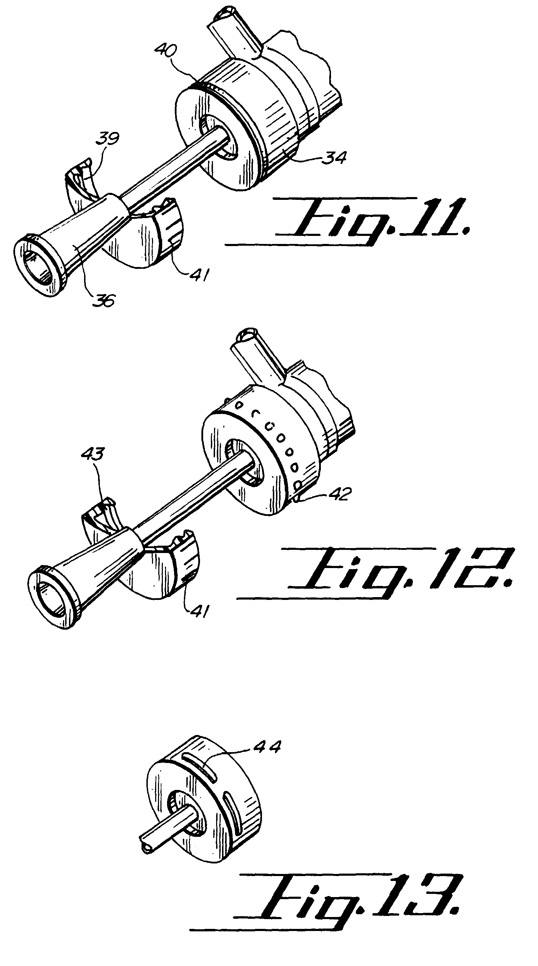

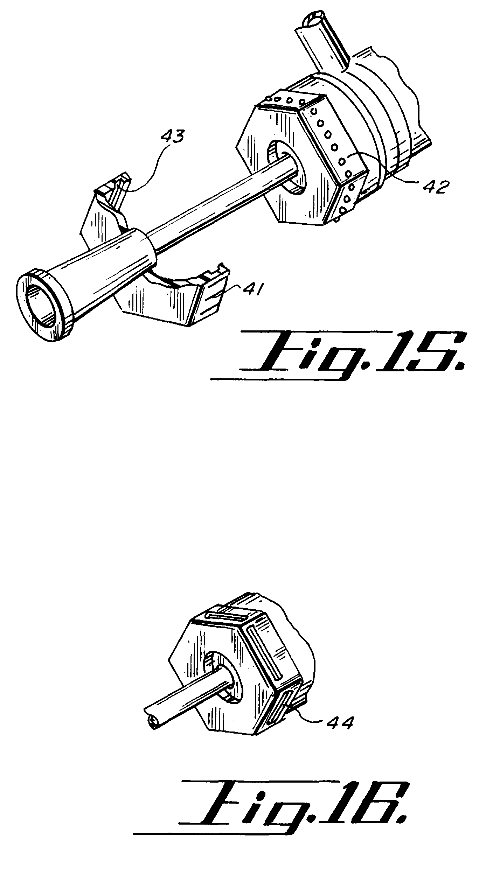

In a device to introduce a catheter into a blood vessel such as for angiography, a catheter introducer is used. As shown in Figure 1, symbol 1 denotes a catheter introducer in general. The catheter introducer 1 includes a sheath section 2, a dilator section 3, a sheath hub 4, a sheath 5, a female hole 6, a dilator hub 7, a dilator 8, a male prism 9, and a side tube 10.

As can be seen in the drawing, the prior art catheter introducer 1 of Figure 1 is composed of the sheath section 2 consisting of the sheath 5 and the sheath hub 4 and the dilator section 3 consisting of the dilator 8 and the dilator hub 7. The catheter introducer 1 has the dilator section 3 set in the sheath section 2, and is inserted into a blood vessel over a guide wire.

Figures 2 and 3 are illustrations showing how the prior art catheter of Figure 1 introducer is used. In the drawings, symbol 11 denotes a three-way stop cock, a guide wire is designated by symbol 12, subcutaneous tissue is designated by symbol 13, and symbol 14 designates a blood vessel. When the catheter introducer is used, a hollow needle (not illustrated) with an inner needle inserted through it is introduced into the blood vessel 14, and after removing the inner needle, the guide wire 12 is inserted through the hollow needle. The hollow needle is subsequently removed, to leave only the guide wire 12 in the blood vessel. Then, as shown in Figure 2, the catheter introducer 1 with the dilator section 3 inserted in it is inserted into the blood vessel 14 with the guide wire 12 operating as a guide, and subsequently, with the sheath 5 of the catheter introducer 1 inserted in the blood vessel 14. As shown in Figure 3, the dilator section 3 and the guide wire 12 are subsequently removed. Then, a catheter is inserted into the sheath 5 which operates to guide the tip of the catheter into the blood vessel 14 to complete the insertion of the catheter into the blood vessel 14. Subsequently, as required, the three-way stop cock 11 may be actuated to supply a medicine, etc. into the blood vessel through the sheath.

If the catheter introducer 1 with the dilator section 3 inserted therein is inserted into the blood vessel 14 with the guide wire operating as a guide while the sheath section 2 is being held and turned clockwise and counterclockwise, the insertion into the blood vessel 14 can be easily achieved. However, to achieve this, any means for mutually arresting the movement of the dilator section 3 and the sheath section 2 is required to prevent the relative rotation between the dilator section 3 and the sheath section 2 as well as preventing the axial displacement thereof.

To prevent the relative rotation and axial displacement of the dilator section 3 with respect to the sheath section 2, several proposals have been disclosed. The proposals are described below. The device as shown in Figure 1 is disclosed in Japanese Patent Publication No. 91-37430 has a female polygonal hole 6 formed in the sheath section 2 and a male prism 9 formed in the dilator section 3. The male prism 9 of the dilator section 3 is inserted in the female polygonal hole 6 of the sheath section 2 to prevent the relative rotation between the dilator section 3 and the sheath section 2. Furthermore, the female polygonal hole 6 has a groove 6a formed and the male prism 9 has a rib 9a formed so that when the male prism 9 of the dilator section 3 is inserted in the female polygonal hole 6 of the sheath section, the rib 9a of the male prism 9 is fitted in the groove 6a of the female polygonal hole 6 for preventing the axial displacement of the dilator section 3 and the sheath section 2.

Figure 4 shows a prior art device disclosed in said Japanese Patent Laid-Open No. 90-289269. The male engaging part 15 of the dilator section 3 and the female engaging part 16 of the sheath section 2 are tapered and hexagonal in section. The tapered engagement can prevent the axial displacement and the relative rotation between the sheath section 2 and the dilator section.

A prior art device shown in Figures 5 and 6 is also disclosed in the same Japanese Patent Laid-Open No. 90-289269. Figure 5 is a front view showing the end of the sheath section 2 with a female taper portion 17 with recesses 18 and Figure 6 is a side view showing the dilator section 3 with a male taper portion 19 with protrusions 20. The male taper portion 19 of the dilator section is inserted into the female taper portion 17 of the sheath section to prevent the axial displacement by the taper portion 17, and to prevent the relative rotation by the engagement between the protrusions 20 and the recesses 18.

The prior art device shown in Figure 7 is shown in Japanese Utility Model Laid-Open No. 89-112849. In this case, a spiral protrusion 21 is formed around the end 2a of the sheath section and a recess 22 to be engaged with the protrusion 21 of the sheath section is formed in the end 3a of the dilator section 3 for fastening the dilator section 3 and the sheath section 2 by screwing the sections together.

US 4,772,266 discloses dilator/sheath assemblies which ensure a stable axial relationship between concentrically superimposed sheath and dilator, whereby the coupling between the dilator and the sheet is obtained by means of a Luer lock needing 90° rotation to lock or unlock, respectively. US 3,406,685 describes a catheter needle assembly with the parts being kept together by Luer locks, and EP 0 258 566 describes a catheter assembly with the parts being connected by a lock with an external radial projection on the catheter part and a lead-in slit and a clearance for the reception of the projection upon rotation on the needle part. EP 008 451 also discloses assembly of catheter part and needle part provided with hub ears and a thread, respectively, by rotation. Although these coupling devices, if correctly coupled, prevent axial displacement as well as rotation in one direction, undesired decoupling may occur upon rotational movement in the 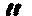 wrong" direction.

### DISCLOSURE OF INVENTION

As described above, several means for engaging the dilator section with the sheath section have been developed, but they are not perfect. In many of the above described devices, the engagement achieved by the interconnection of the tapered portions is weak and unstable and may cause disengagement during use.

The use of protrusions and recesses shown in Figures 5 and 6 overcomes this disadvantage to some extent. However, for reliable engagement between protrusions and recesses, the length of engagement between the elements must be long enough to prevent disengagement therebetween. As a result, as shown in Figure 8, the depth D from the end of the sheath section to the check valve must be long, and in this situation, it is difficult to insert a guide wire or catheter which may be bent or precurved at the tip into the sheath, since the tip of the catheter deviates from the center of the check valve as shown in Figure 8.

The device shown in Figure 7 overcomes this disadvantage, but screwing is required for engaging the dilator section with the sheath section which is a troublesome action.

The present invention has been created to solve the problems of the conventional catheter introducers described above, and an object of the present invention is to provide a catheter introducer which allows the dilator section to be engaged with and disengaged from the sheath section promptly and smoothly.

To achieve the above object, the catheter introducer of the present device has a flange formed at the end of the dilator section. The flange covers the end of the sheath hub in engagement with the dilator section. The dilator section also has a groove formed in the internal circular portion of the sheath hub and a protrusion to be engaged with the groove may be formed on the external circular portion of the sheath hub or the internal circular portion of the flange.

In the catheter introducer composed as described above, when the catheter introducer provided with the dilator section is inserted into the blood vessel, the protrusion formed on the sheath section or the dilator section is engaged with the groove formed on the other of the dilator section or the sheath section to integrate the dilator section and the sheath section. So, even if the catheter introducer is inserted into the blood vessel while the sheath section is being held and turned clockwise and counterclockwise, it does not happen that the dilator section and the sheath section are rotated relatively or displaced axially.

For disengaging the dilator section from the sheath section, both can be disengaged by slightly inclining or canting the dilator hub with respect to the sheath hub while pulling on the dilator hub.

### BRIEF DESCRIPTION OF DRAWINGS

Figure 1 is a side view showing a prior art catheter introducer;
Figure 2 is a side view of the prior art catheter introducer shown in Figure 1, showing how the catheter introducer is used;
Figure 3 is a side view of the catheter introducer further showing how the catheter introducer is used;
Figure 4 is a partial perspective view showing a portion of a dilator inserted into a sheath hub of a prior art catheter introducer;
Figure 5 is a partial front view showing the sheath hub of another prior art catheter introducer;
Figure 6 is a partial side view showing the dilator section of the prior art catheter introducer shown in Figure 5;
Figure 7 is a cross-sectional view showing the threaded engagement between the dilator section and a sheath section of a prior art catheter introducer;
Figure 8 is an illustration showing where a bent or precurved guide wire or catheter is inserted into the sheath of a catheter introducer of the type shown in Figures 5 and 6;
Figure 9 is an enlarged cross-sectional view showing a catheter introducer as an example of the present invention;
Figure 10 is an enlarged perspective view showing the catheter introducer of Figure 9;
Figure 11 is an enlarged partial perspective view showing an important portion of a variation of the catheter introducer of Figure 9;
Figure 12 is an enlarged partial perspective view showing an important portion of another embodiment of a catheter introducer of the present invention;
Figure 13 is an enlarged perspective view showing an important portion of another embodiment of a sheath hub of a catheter introducer of the present invention;
Figure 14 is an enlarged perspective view of another embodiment of the present invention;
Figure 15 is an enlarged partial perspective view of an alternate embodiment of the present invention showing the dilator and sheath hubs; and
Figure 16 is an enlarged partial perspective view of the sheath hub of an alternate embodiment of the present invention.

### BEST MODE FOR CARRYING OUT THE INVENTION

As can be seen in the drawings, in the catheter introducer of the present invention, the flange 37 with the circular protrusion 39 formed on its internal circular portion is provided at the distal end of the dilator hub 36, and the groove 40 to be engaged with the circular protrusion 39 is formed in the external circular portion of the proximal end of the sheath hub 34 of the sheath section 32. The flange 37 preferably has a plurality of slits 41 thereon formed in the axial direction. It is preferred that the groove 40 is formed to be completely engaged with the circular protrusion 39. It is also possible that the circular protrusion 39 is formed on the external circular portion of the sheath hub 34 of the sheath section 32 while the groove 40 may be formed in the internal circular portion of the flange 37.

When the catheter introducer is inserted into the blood vessel, as illustrated in Figures 2 and 3, the dilator 38 of the dilator section 33 is an elongate and tubular member which is inserted into the sheath 35 of the sheath section 32, and the dilator hub 36 is pressed to the sheath hub 34 in the axial direction. Since both the flange 37 and the dilator hub 36 are made of a soft plastic material, the flange 37 covers the external circular portion of the sheath hub 34 in the state of engagement, and the circular protrusion 39 on the flange 37 is engaged with the groove 40 of the sheath hub 34. As a result, the dilator section 33 and the sheath section 32 are integrated, and even if only the sheath section 32 or the dilator section 33 is held and turned clockwise and counterclockwise, it does not happen that the dilator section 33 and the sheath section 32 rotate relatively or are displaced in the axial direction. Therefore, the insertion of the catheter introducer 31 into the blood vessel can be achieved smoothly and easily.

To disengage the dilator section 33 from the sheath section 32, if the proximal end of the dilator hub 36 is lightly pressed or canted in the direction perpendicular to the axial direction, the protrusion 39 of the flange 37 is disengaged from the groove 40 of the sheath 34.

Figures 9-11 are illustrative of a preferred form of the present invention. The catheter introducer is generally referred to herein as catheter introducer 31. The catheter introducer 31 includes a sheath section 32 and a dilator section 33. The sheath section 32 includes a sheath hub 34 having circumferential groove 40 thereon and an elongate sheath 35. The dilator section 33 includes a dilator hub 36 with a flange 37 having a circumferential circular protrusion 39 thereon and an elongate dilator portion 38. Therefore, the insertion of the catheter introducer into the blood vessel can be achieved smoothly and easily.

As shown in Figures 9-16 in the catheter introducer 31 of the present invention, a flange 37 is preferably provided around the dilator hub 36 of the dilator section 33 to cover the sheath hub 34 in the state of engagement and a protrusion 39 and a groove 40 to be engaged with each other are formed on or in the flange 37 and the sheath hub 34. So, even if the sheath section 32 of the catheter introducer 31 is held and turned clockwise and counterclockwise for insertion of the catheter into a blood vessel, it does not happen that the dilator section 33 and the sheath section 32 rotate relatively or are displaced in the axial direction and the insertion of the catheter introducer 31 into the blood vessel can be achieved easily and reliably as an excellent effect of the present device.

To disengage the dilator section 33 from the sheath section 32, if the end on the side of the dilator section 33 opposite to the flange 37 of the dilator hub is lightly pressed or canted in the direction perpendicular to the axial direction, the protrusion 39 of the flange is disengaged from the groove 40 of the sheath hub.

Figure 12 is an enlarged partial perspective view showing another example of the present invention. In this embodiment, a plurality of protrusion dots 42 are formed on the external circular portion of the sheath hub 34, and the groove 43 is formed in the internal circular portion of the flange 37 to be engaged with the protrusion dots 42.

Figure 13 shows an embodiment of the present invention where protrusion strips 44 are formed along the circumference of the sheath hub 34 instead of the protrusion dots 42 of the above embodiment. In these embodiments, slits 41 are formed in the flange 37.

As can be seen in Figures 14-16, in the catheter introducer 31 of the present embodiment, a regularly hexagonal flange 37 with the continuous protrusion 39 on the internal circular portion is provided at the end of the dilator section 33 and the sheath hub 34 of the sheath section 32 is formed to be regularly hexagonal with the groove 40 in its external circular portion to allow the hexagonal sheath hub 34 to be fitted in the internal circular portion of the flange 37 at the end of the dilator section. If the sheath hub is fitted in the internal circular portion of the flange 37 at the end of the dilator section 33, the protrusion 39 on the internal circular portion of the flange is fitted in the groove 40 of the sheath hub 34 to prevent the axial displacement of the dilator section 33 and the sheath section 32. Furthermore, since the sheath hub 34 is fitted in the hexagonal internal circular portion of the flange 37, the relative rotation between the dilator section 33 and the sheath section 32 does not occur. Moreover, plural slits 41 are formed in the external circular portion of the flange 37 in the axial direction conveniently for covering the sheath hub 34 with the flange 37 in the state of engagement.

Figure 15 is an enlarged perspective view showing another embodiment of the present invention. In this embodiment, multiple protrusion dots 42 are formed on the external circular portion of the sheath hub 34 and the groove 43 is formed in the internal circular portion of the flange 37 to be engaged with the protrusion dots 42.

Figure 16 shows an embodiment where protrusion strips 44 are formed instead of the protrusion dots 42 disclosed in the above embodiment. In these embodiments described above, slits 41 are preferably formed in the flange 37.

With this embodiment, it is also possible to form the protrusion 39 on the external circular portion of the sheath hub 34 of the sheath section and to form the groove 40 in the internal circular portion of the flange 37.

In these embodiments, the internal circular portion of the flange 37 and the sheath hub 32 are formed to be regularly hexagonal but the form is not limited to hexagon. It is only preferred to be a regular polygon which includes a square or pentagon.

When the catheter introducer 31 of the present invention is inserted into the blood vessel in the manner shown in Figures 2 and 3, the dilator 38 of the dilator section 33 is inserted into the sheath 35 of the sheath section 31, and the dilator hub 36 is pressed to the sheath hub 34 in the axial direction. Since both the flange 37 and the dilator hub 36 are made of a soft plastic material, the flange 37 covers the external circular portion of the sheath hub 34 in the state of engagement, and the protrusion 39 is engaged with the groove 40 of the sheath hub. As a result, the dilator section 33 and the sheath section 32 are integrated and even if only the sheath section or the dilator section is held and turned clockwise and counterclockwise, it does not happen that the dilator section 33 and the sheath section 32 rotate relatively or are displaced in the axial direction.

## Claims

1. A catheter introducer (31) comprising:
a sheath section (32) having distal and proximal end portions;
a generally cylindrical sheath hub (34) formed on said proximal end portion of said sheath section, said sheath hub including an outer circumference and a longitudinal passageway extending therethrough;
a generally elongate and tubular sheath member (35) formed on said distal end portion of said sheath section, a longitudinal passageway extending therethrough and said passageway extending in flow communication with said passageway of said sheath hub;
a dilator section (33) having distal and proximal end portions thereof and said dilator section being operatively associated with said sheath section in use;
a generally cylindrical dilator hub (36) formed on said proximal end portion of said dilator section, said dilator hub including a flange means (37) extending from a distal portion thereof and said flange being sized to receive at least a portion of said sheath hub therewith; and
means for locking (39,40) said sheath hub (34) on said dilator hub (36) wherein said means for locking includes a protrusion or channel and one of said protrusion (39) or channel (40) is formed on said outer circumference of said sheath hub and the other of said protrusion or channel is formed on said flange member, characterized in that said protrusion and said channel being formed to interlock such that rotational and longitudinal movement of said sheath hub and said dilator hub is prevented when said protrusion and said channel are interlocked and said protrusion and channel are formed to unlock when the proximal end of the dilator hub (36) is lightly pressed or canted in the direction perpendicular to the axial direction to release said sheath hub from said dilator hub, and whereby said flange means and said dilator hub are made of a soft plastic material.

2. The catheter introducer of claim 1 wherein said flange member (37) includes an inner surface thereon and said inner surface includes the other of said protrusion (39) or channel (40) thereon.

3. The catheter introducer of claim 2 wherein said inner surface of said flange member (37) includes a channel (43) therein and said outer circumference of said sheath hub (34) includes a protrusion (42, 44) thereon.

4. The catheter introducer of claim 3 wherein said protrusion extends along the entire circumference of said outer circumference of said sheath hub.

5. The catheter introducer of claim 3 wherein said flange member is sized to substantially extend along said outer circumference of said sheath hub and said flange includes a plurality of elongate slits (41) therein.

6. The catheter introducer of claim 1 wherein said outer circumference of said sheath hub (34) is formed as a regular polygon and said flange member is formed to include an interior surface having a regular polygonal surface which is complementary to said outer circumference of said sheath hub.

7. The catheter introducer of claim 1 wherein said outer circumference of said sheath hub (34) includes said channel (40) thereon and said flange member (37) includes said protrusion (39) thereon.

8. The catheter introducer of claim 7 wherein said flange member (37) includes an inner surface thereon and said protrusion (39) extends substantially therealong.

9. The catheter introducer of claim 8 wherein said protrusion is formed of a plurality of protrusions (42, 44) aligned along said inner surface of said dilator hub.

10. The catheter introducer of claim 7 wherein said channel (40) extends along the entire circumference of said outer circumference of said sheath hub (34).

11. The catheter introducer of claim 7 wherein said flange member includes a plurality of slits (41) thereon.

## Patentansprüche

1. Kathetereinführer (31), aufweisend:
ein Hülsenstück (32) mit einem distalen und einem proximalen Endabschnitt;
eine allgemein zylindrische Hülsennabe (34), die an dem proximalen Endabschnitt des Hülsenstücks angeformt ist, wobei die Hülsennabe einen äußeren Umfang und einen longitudinalen, sich hindurch erstreckenden, Durchgang umfaßt;
ein allgemein längliches und röhrenförmiges Hülsenelement (35), das an dem distalen Endabschnitt des Hülsenstücks geformt ist, einen longitudinalen Durchgang, der sich hindurch erstreckt und sich in Fließverbindung mit dem Durchgang der Hülsennabe erstreckt;
ein Dilatorstück (33), das einen distalen und einen proximalen Endabschnitt desselben aufweist, und wobei das Dilatorstück im Gebrauch operativ mit dem Hülsenstückabschnitt verbunden ist;
eine allgemein zylindrische Dilatornabe (36), die an dem proximalen Endabschnitt des Dilatorstücks angeformt ist, wobei die Dilatornabe einen Flansch (37) umfaßt, der sich vom distalen Abschnitt aus erstreckt, und wobei der Flansch so bemessen ist, daß er mindestens einen Abschnitt der Hülsennabe aufnimmt; und
Mittel zum Verriegeln (39, 41) der Hülsennabe (34) an die Dilatornabe (36), wobei die Mittel zum Verriegeln einen Vorsprung oder Kanal umfassen und entweder der Vorsprung (39) oder der Kanal (40) an dem äußeren Umfang der Hülsennabe geformt ist und der andere von ihnen, der Vorsprung oder der Kanal, an dem Flanschelement geformt ist, dadurch gekennzeichnet, daß der Vorsprung und der Kanal so ausgebildet sind, daß sie sich so verriegeln, daß eine drehende und longitudinale Bewegung der Hülsennabe und der Dilatornabe verhindert wird, wenn der Vorsprung und der Kanal miteinander verriegelt sind, und daß der Vorsprung und der Kanal so ausgebildet sind, daß sie sich entriegeln, wenn das proximale Ende der Dilatornabe (36) leicht angedrückt oder in Richtung senkrecht zur achsialen Richtung gekippt wird, um die Hülsennabe von der Dilatornabe zu lösen, und wobei das Flanschmittel und die Dilatornabe aus einem weichen Kunststoff hergestellt sind.

2. Kathetereinführer nach Anspruch 1, bei dem das Flanschelement (37) eine innere Oberfläche darauf aufweist, und die innere Oberfläche das andere Mittel, den Vorsprung (39) oder den Kanal (40), aufweist.

3. Kathetereinführer nach Anspruch 2, bei dem die innere Oberfläche des Flanschelementes (37) einen Kanal (43) aufweist, und der äußere Umfang der Hülsennabe (34) einen darauf befindlichen Vorsprung (42) aufweist.

4. Kathetereinführer nach Anspruch 3, bei dem sich der Vorsprung entlang des gesamten Umfangs des äußeren Umfangs der Hülsennabe erstreckt.

5. Kathetereinführer nach Anspruch 3, bei dem das Flanschelement so bemessen ist, daß es sich im wesentlichen entlang des äußeren Umfangs der Hülsennabe erstreckt und der Flansch eine Vielzahl von darin angebrachten Längsschlitzen (41) aufweist.

6. Kathetereinführer nach Anspruch 1, bei dem der äußere Umfang der Hülsennabe (34) als regelmäßiges Polygon ausgebildet ist und das Flanschelement so geformt ist, daß es eine innere Oberfläche mit einer regelmäßigen polygonalen Oberfläche aufweist, die dem äußeren Umfang der Hülsennabe komplementär ist.

7. Kathetereinführer nach Anspruch 1, bei dem der äußere Umfang der Hülsennabe (34) den Kanal (40) darauf aufweist und das Flanschelement (37) den Vorsprung darauf aufweist.

8. Kathetereinführer nach Anspruch 7, bei dem das Flanschelement (37) eine innere Oberfläche darauf aufweist und der Vorsprung (39) sich im wesentlichen entlang derselben erstreckt.

9. Kathetereinführer nach Anspruch 8, bei dem der Vorsprung aus einer Vielzahl von Vorsprüngen (42, 44) gebildet ist, die entlang der inneren Oberfläche der Dilatornabe gereiht sind.

10. Kathetereinführer nach Anspruch 7, bei dem der Kanal (40) sich entlang des gesamten Umfangs des äußeren Umfangs der Hülsennabe (34) erstreckt.

11. Kathetereinführer nach Anspruch 7, bei dem das Flanschelement eine Vielzahl von Schlitzen (41) darauf aufweist.

## Revendications

1. Introducteur de cathéter (31) comprenant :
une section de gaine (32) présentant des portions d'extrémité distale et proximale;
un embout femelle de gaine (34) cylindrique d'une manière générale, formé sur la portion d'extrémité proximale susdite de la section de gaine. ledit embout femelle de gaine comprenant une circonférence externe et un passage longitudinal la traversant;
un élément de gaine allongé et tubulaire d'une manière générale, formé sur la portion d'extrémité distale susdite de la section de gaine, un passage longitudinal le traversant, ce passage étant en communication pour écoulement d'un fluide avec le passage de l'embout femelle de gaine;
une section de dilatateur (33) présentant des portions d'extrémité distale et proximale, la section de dilatateur étant associée, fonctionnellement, à la section de gaine en situation d'utilisation;
un embout mâle de dilatateur (36) cylindrique, d'une manière générale, formé sur la portion d'extrémité proximale de la section de dilatateur, l'embout mâle de dilatateur présentant un collet (37) partant d'une portion distale de celui-ci, le collet étant dimensionné de façon à recevoir au moins une portion de l'embout femelle de gaine; et;
un moyen pour verrouiller (39, 40) l'embout femelle de gaine (34) sur l'embout mâle de dilatateur (36), ce moyen comprenant une excroissance ou un canal, et l'un ou l'autre de l'excroissance (39) ou du canal (40) est formé sur la circonférence extérieure de l'embout femelle de gaine et l'autre de l'excroissance ou du canal est formé sur le collet, introducteur de cathéter caractérisé en ce que l'excroissance et le canal sont formés pour assurer un verrouillage réciproque de façon telle que le mouvement rotatif et longitudinal de l'embout femelle de gaine et de l'embout mâle de dilatateur soit empêché lorsque l'excroissance et le canal sont verrouillés réciproquement et en ce que l'excroissance et le canal sont formés de manière à se déverrouiller lorsque l'extrémité proximale de l'embout mâle de dilatateur (36) est légèrement pressée ou basculée à la perpendiculaire de la direction axiale, de façon à dégager l'embout femelle de gaine de l'embout mâle de dilatateur, introducteur de cathéter dont le collet et l'embout mâle de dilatateur sont faits dans une matière plastique tendre.

2. Introducteur de cathéter selon la revendication 1, dans lequel le collet (37) comprend une surface interne et la surface interne comprend l'un ou l'autre de l'excroissance (39) ou du canal (40).

3. Introducteur de cathéter selon la revendication 2, dans lequel la surface interne du collet (37) comprend un canal (43) et où la circonférence externe de l'embout femelle de gaine (34) comprend une excroissance (42, 44).

4. Introducteur de cathéter selon la revendication 3, dans lequel l'excroissance s'étend sur tout le pourtour de la circonférence externe de l'embout femelle de gaine.

5. Introducteur de cathéter selon la revendication 3, dans lequel le collet est dimensionné de façon à s'étendre quasiment sur la circonférence externe de l'embout femelle de gaine et où le collet comprend plusieurs encoches allongées (41).

6. Introducteur de cathéter selon la revendication 1, dans lequel la circonférence externe de l'embout femelle de gaine (34) adopte une forme de polygone régulier et où le collet est formé de façon à inclure une surface interne présentant une surface polygonale régulière complémentaire de la circonférence externe de l'embout femelle de gaine.

7. Introducteur de cathéter selon la revendication 1, dans lequel la circonférence externe de l'embout femelle de gaine (34) comprend le canal (40) et où le collet (37) comprend l'excroissance (39).

8. Introducteur de cathéter selon la revendication 7, dans lequel le collet (37) comprend une surface interne et où l'excroissance (39) s'étend quasiment le long de cette dernière.

9. Introducteur de cathéter selon la revendication 8, dans lequel l'excroissance consiste en une pluralité d'excroissances (42, 44) alignées le long de la surface interne de l'embout mâle de dilatateur.

10. Introducteur de cathéter selon la revendication 7, dans lequel le canal (40) s'étend sur tout le pourtour de la circonférence externe de l'embout femelle de gaine (34).

11. Introducteur de cathéter selon la revendication 7, dans lequel le collet comprend plusieurs encoches (41).
